# EUROPÄISCHE PATENTANMELDUNG

(11) **EP 1 759 633 A1**
(43) Veröffentlichungstag der Anmeldung: **07.03.2007**
(21) Anmeldenummer: 05019055.2
(22) Anmeldetag: 01.09.2005
(51) Int. Cl.: A61B 5/05

(54) **Anordnung zum Aufnehmen von Körperflüssigkeiten sowie Herstellungsverfahren hierfür**

(71) Anmelder: F.HOFFMANN-LA ROCHE AG, 4070 Basel (CH); Roche Diagnostics GmbH, 68305 Mannheim (DE)
(72) Erfinder: Haar, Hans-Peter, Dr., 69168 Wiesloch (DE); Calasso, Irio Guiseppe, Dr., 6415 Arth (CH); Fuerst, Otto, Dr., 68519 Viernheim (DE)
(74) Vertreter: Pfiz, Thomas

(57) **Zusammenfassung**

Bei einer Anordnung zum Aufnehmen von Körperflüssigkeiten mit einem Probenentnahmeelement (10), das einen Sammelbereich (12) zum Sammeln von durch einen Einstich erhaltener Körperflüssigkeit aufweist, wird vorgeschlagen, dass der Sammelbereich durch einen als Kapillare langgestreckten, über Seitenöffnungen (28,30) an dem Probenentnahmeelement (10) beidseitig offenen Längsschlitz (12) gebildet ist.

## Beschreibung

Die Erfindung betrifft eine Anordnung zum Aufnehmen von Körperflüssigkeiten wie Blut, mit einem vorzugsweise mit einem Stechorgan zum Einstechen in ein Körperteil versehenen Probenentnahmeelement, das einen Sammelbereich zum Sammeln von durch einen Einstich erhaltener Körperflüssigkeit aufweist. Die Erfindung betrifft weiter ein Verfahren zur Herstellung eines solchen Probenentnahmeelements.

In einer früheren Anmeldung PCT/EP2005/002357 der Anmelder ist eine Anordnung zum Sammeln von Körperflüssigkeit für Analysezwecke, speziell zur Blutglucose-Konzentrationsbestimmung beschrieben. Daraus geht ein Stechelement mit einer Sammelzone zum Aufnehmen von Körperflüssigkeit hervor, wobei die Sammelzone durch Querbohrungen gebildet sein kann. Der Flüssigkeitstransfer kann damit quer zur Stechrichtung auf ein in fluidischen Kontakt gebrachtes Probenaufnahmeelement erfolgen, so dass ein kapillarer Transport über makroskopische Strecken mit hohem Totvolumen vermieden werden kann.

Ausgehend hiervon liegt der Erfindung die Aufgabe zugrunde, die im Stand der Technik bekannten Systeme weiter zu entwickeln und eine Anordnung der eingangs angegebenen Art im Sinne einer verbesserten Probenaufnahme zu optimieren, wobei ein Erfindungsziel auch in einer vereinfachten Herstellung besteht.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, eine möglichst zuverlässige kapillare Probenaufnahme durch einen beidseitig offenen Kapillarschlitz zu erreichen. Dementsprechend wird erfindungsgemäß vorgeschlagen, dass der Sammelbereich durch einen als Kapillare langgestreckten, über Seitenöffnungen an dem Probenentnahmeelement beidseitig offenen Längsschlitz gebildet ist. Dadurch kann eine rasche Probenaufnahme von beiden Seiten in einem einheitlichen Volumen gewährleistet werden, wobei aufgrund der Kapillarwirkung keine äußere Einwirkung erforderlich ist. Besonders bevorzugt verläuft der Schlitz in Längs- bzw. Stechrichtung eines als Stechelement ausgebildeten Probenentnahmeelements. Denkbar ist aber auch eine Probenaufnahme nach Erzeugung eines Einstichs mittels eines gesonderten Stechapparats. Im Vergleich zu röhrenförmigen oder halboffenen Kapillaren wird durch die offene Schlitzgestaltung auch die Gefahr von Blockierungen durch Gewebebestandteile weiter verringert. Durch die beidseitige Öffnung kann zudem das weitere Probenhandling wesentlich verbessert werden, wobei ein weitgehend totvolumenfreier Transfer erreichbar ist.

Gemäß einer bevorzugten Ausgestaltung der Erfindung ist die Länge des Längsschlitzes so bemessen, dass in einer Sammelstellung des Probenentnahmeelements der Längsschlitz teilweise innerhalb und teilweise außerhalb des Körperteils ist. Auf diese Weise kann bei der Flüssigkeitsaufnahme eine Entlüftungsfunktion des Schlitzes erreicht werden, und es steht zusätzliches Sammelvolumen zur Verfügung. In dieser Hinsicht ist es vorteilhaft, wenn der Längsschlitz beim Sammeln der Körperflüssigkeit einen in die Haut des Körperteils hineinragenden distalen Aufnahmeabschnitt und einen außerhalb der Haut befindlichen proximalen Entlüftungsabschnitt aufweist.

Vorteilhafterweise besitzt der Längsschlitz eine Länge von 0,5 bis 4 mm, vorzugsweise von 1 bis 2 mm und eine Breite von weniger als 500 µm, vorzugsweise weniger als 100 µm. Weiter ist es von Vorteil, wenn der Längsschlitz im Abstand vorzugsweise von etwa 50 bis 200 µm zu einer das Stechorgan bildenden distalen Spitze des Probenentnahmeelements angeordnet ist.

Um den bei einem Einstich empfundenen Schmerz zu reduzieren und gleichzeitig ein hinreichendes Aufnahmevolumen zu schaffen, kann das Probenentnahmeelement im Bereich des Längsschlitzes einen im Querschnitt verjüngten distalen Schaftabschnitt und einen im Querschnitt erweiterten proximalen Schaftabschnitt aufweisen.

Ein auch hinsichtlich der Analyse weiter integriertes System sieht ein über den Sammelbereich mit Körperflüssigkeit beaufschlagbares, vorzugsweise mit einem Testfeld für einen Analyten in der Körperflüssigkeit versehenes Probenempfangselement vor. Hierbei ist es für einen schnellen und möglichst verlustfreien Flüssigkeitstransfer auf ein zuvor getrenntes Empfangselement von besonderem Vorteil, wenn eine Seitenöffnung als Auslass des Längsschlitzes mit dem Probenempfangselement in fluidischen Kontakt bringbar ist.

Eine weitere Verbesserung in dieser Richtung wird dadurch erreicht, dass der Längsschlitz in der Sammelstellung des Probenentnahmeelements von dem Probenempfangselement räumlich und/oder fluidisch getrennt, und in einer Transferstellung des Probenentnahmeelements mit dem Probenempfangselement zum Transfer von Körperflüssigkeit über eine Seitenöffnung gekoppelt ist.

Hierbei ist es auch von Vorteil, wenn die dem Auslass gegenüberliegende Seitenöffnung des Längsschlitzes in einer Transferstellung an einen Aktuator zum Transfer der Körperflüssigkeit auf das Probenempfangselement angeschlossen ist.

Vorteilhafterweise wirkt der Aktuator über pneumatische und/oder mechanische Verdrängungsmittel auf die in dem Längsschlitz befindliche Körperflüssigkeit ein, wobei ein mechanischer Aktuator durch eine gegen den Längsschlitz deformierbare Membran gebildet sein kann. Günstig ist es auch, wenn der Aktuator eine an die von dem Probenempfangselement abgewandte Seitenöffnung des Längsschlitzes ankoppelbare Druckluftpassage aufweist.

Für einen möglichst vollständigen Flüssigkeitstransfer ist es vorteilhaft, wenn das Probenempfangselement eine größere Kapillarattraktion für die Körperflüssigkeit als der Längsschlitz aufweist.

Eine weitere vorteilhafte Ausgestaltung sieht vor, dass das Probenentnahmeelement in einer Führung relativ zu dem Probenempfangselement beweglich gelagert ist.

Weitere Gebrauchsvorteile für den Benutzer lassen sich dadurch erzielen, dass mehrere Probenentnahmeelemente in einem ersten Magazin und mehrere Probenempfangselemente in einem zweiten Magazin bevorratet sind, wobei die Magazine als gesonderte Einheiten zur paarweisen Kopplung der Probenentnahmeelemente und Probenempfangselemente miteinander verbindbar sind.

Eine vorteilhafte Realisierungsmöglichkeit besteht darin, dass eine Mehrzahl von Probenentnahmeelementen in einem Magazin, vorzugsweise einem Trommelmagazin axial ausstoßbar angeordnet sind, und dass zugeordnete Probenempfangselemente vorzugsweise in Durchstoßkammern dem Magazin in Ausstoßrichtung vorgeordnet sind.

Gegenstand der Erfindung ist auch ein tragbares Blutanalysegerät mit mindestens einer vorzugsweise als Einmalartikel ausgebildeten erfindungsgemäßen Sammelanordnung.

In verfahrensmäßiger Hinsicht wird die eingangs genannte Aufgabe dadurch gelöst, dass in dem Probenentnahmeelement ein beidseitig offener Längsschlitz als Sammelbereich für die Körperflüssigkeit durch Laserschneiden gebildet wird.

Hierbei ist es günstig, wenn die Begrenzungsfläche des Längsschlitzes beim Laserschneiden hydrophiliert wird.

Eine weitere Verbesserung sieht vor, dass die Laserenergie und/oder die Verfahrgeschwindigkeit des Laserstrahls beim Laserschneiden positionsabhängig geregelt wird, so dass unterschiedliche Materialstärken besser berücksichtigt werden können.

Vorteilhafterweise beträgt die Bearbeitungszeit während des Laserschneidens weniger als 2 s, vorzugsweise etwa 1 s.

Auch für eine erhöhte Kapillarwirkung ist es von Vorteil, wenn die Begrenzungskanten des Längsschlitzes mit einem Kantenwinkel von weniger als 100°, vorzugsweise etwa 90° gebildet werden.

Eine weitere vorteilhafte Verfahrensweise sieht vor, dass an dem Probenentnahmeelement ein scharfes Stechorgan durch Schleifen erzeugt wird, so dass das Stechorgan durch mindestens einen Flachschliff begrenzt wird. Hierbei ist es auch von Vorteil, wenn ein Draht oder ein flaches Band als Ausgangsmaterial bearbeitet wird.

Im Folgenden wird die Erfindung anhand der in der Zeichnung schematisch dargestellten Ausführungsbeispiele näher erläutert. Es zeigen
- Fig. 1: ein Handgerät zur Blutzuckermessung in einer perspektivischen Ansicht;
- Fig. 2: ein Stechelement mit einem Längsschlitz zur Blutaufnahme in einer perspektivischen Ansicht;
- Fig. 3: ein integriertes System mit Stechelement und Testelement in verschiedenen Prozessschritten in der Perspektive;
- Fig. 4: einen Fig. 3 entsprechenden Messablauf im Axialschnitt;
- Fig. 5: ein integriertes Magazinsystem für ein Blutzuckermessgerät in einer Explosionsdarstellung;
- Fig. 6: den Messablauf beim Einsatz des Magazinsystems nach Fig. 5 im Längsschnitt;
- Fig. 7: eine ausschnittsweise Vergrößerung der Fig. 6c.

Die in der Zeichnung dargestellte Messanordnung 1 umfasst mindestens ein Probenentnahmeelement 10 mit einem als Längsschlitz 12 ausgebildeten Sammelbereich zum Sammeln von an einer Einstichstelle aus einem Körperteil 14 erhaltener Körperflüssigkeit und ein mit dem Längsschlitz in fluidische Verbindung bringbares Probenempfangselement 16 zum Blutzuckernachweis.

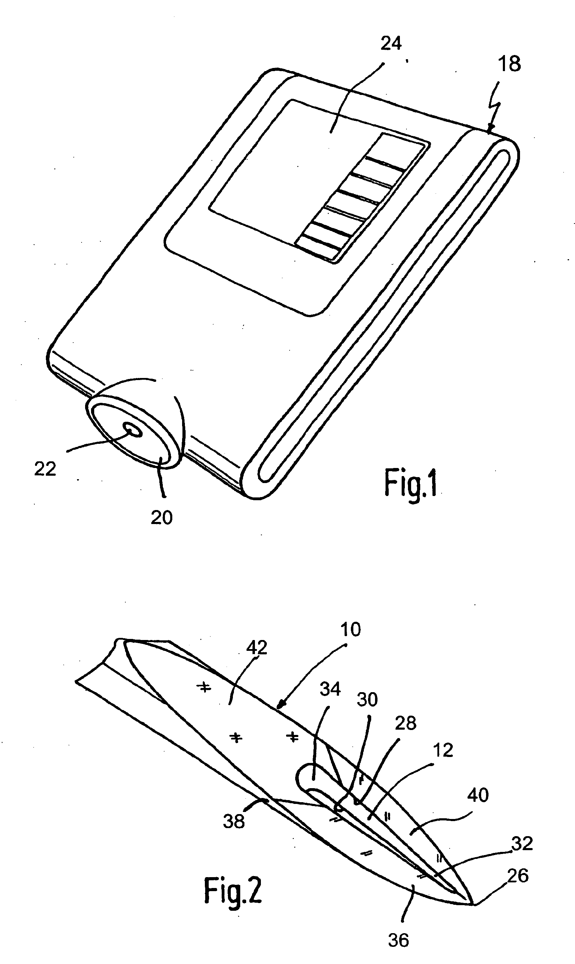

Fig. 1 zeigt ein portables Blutzuckermessgerät 18 für den Einsatz einer solchen Messanordnung 1 für das so genannte "Spot-Monitoring", d.h. die Selbstbestimmung der Blutzuckerkonzentration zu einer gegebenen Zeit durch einen Probanden. Zu diesem Zweck weist das Gerät 18 eine nach innen konische Auflage 20 zur Fingerpositionierung über einer Durchstechöffnung 22 für das Probenentnahme- bzw. Stechelement 10. Die einzelnen Gerätekomponenten werden in einem vollautomatischen Messablauf aktiviert, so dass der Benutzer schließlich auf einer Anzeige 24 einen Messwert über seinen momentanen Blutglukosespiegel erhält, ohne dass eine aufwändige Handhabung erforderlich wäre. Generell können solche Messungen auch an anderen Körperteilen, beispielsweise im weniger schmerzempfindlichen Arm- oder Bauchbereich vorgenommen werden, wobei als Körperflüssigkeit für die Probennahme neben Kapillarblut aus der Haut auch Gewebeflüssigkeit oder Mischungen davon in Frage kommen.

Wie aus Fig. 2 zu ersehen, erstreckt sich der Längsschlitz 12 in Längsrichtung des schaftförmigen Stechelements 10, beispielsweise über eine Länge von 1 bis 2 mm bei einer Breite von 100 bis 200 µm. Der Abstand zu einer das Stechorgan bildenden Spitze 26 kann etwa der Schlitzbreite entsprechen. Auf diese Weise wird eine Kapillaraufnahme gebildet, in welche die beim Einstich in dem Körperteil 14 zugängliche Körperflüssigkeit aufgrund von Kapillarwirkung selbsttätig einströmt.

Die Flüssigkeitsaufnahme erfolgt beidseitig über die einander gegenüberliegenden Seitenöffnungen 28, 30 des Längsschlitzes 12. Hierbei ist die Schlitzlänge in Stechrichtung so bemessen, dass ein distaler Schlitzabschnitt 32 in die Haut des Körperteils 14 hineinragt und ein proximaler Schlitzabschnitt 34 sich außerhalb der Haut befindet. Zur Schmerzverringerung kann der in den Körper eingreifende distale Schaftabschnitt 36 "dünn", d.h. im Querschnitt verjüngt ausgeführt sein, während der außerhalb des Körpers verbleibende proximale Schaftabschnitt 38 demgegenüber einen erweiterten Querschnitt bzw. eine vergleichsweise größere Dicke aufweist, um eine hinreichend große Flüssigkeitsmenge sammeln zu können. Beispielsweise kann die Flüssigkeitsmenge entsprechend dem Schlitzvolumen 10 bis 20 Nanoliter betragen, wobei das im Körper befindliche Teilvolumen des Längsschlitzes 12 beispielsweise einen Volumenanteil von 20% umfasst.

Bei der Herstellung solcher Stechelemente 10 wird zunächst ein Draht als Ausgangsmaterial in einer definierten Ausrichtung durch Schleifprozesse bearbeitet, wobei die unterschiedlich abgewinkelten Schliffflächen 40, 42 erzeugt werden. Sodann wird in dieser Flächenstruktur zweckmäßig in derselben Bearbeitungsstation unter Beibehaltung der Drehausrichtung mittels eines geeignet positionierten Lasers der Längsschlitz 12 eingebracht. Ein wichtiges Produktionsdetail liegt darin, dass die Laserenergie und/oder die Verfahrgeschwindigkeit des Laserstrahls in Abhängigkeit von der Schneidposition geregelt werden; damit lassen sich zulaufende Öffnungen gestalten oder auch unterschiedliche Materialdicken kompensieren.

Für den Schlitzdurchbruch sind nur wenige Bewegungen des Laserstrahls nötig, und die Bearbeitungszeit kann für eine Massenfertigung ausreichend kurz gehalten werden, beispielsweise im Bereich von 1 sec. Ebenso können die mechanischen Toleranzen gering gehalten werden, beispielsweise im Bereich von 10 µm, wobei Wandsteilheiten von nahezu 90° erreichbar sind. Zudem können durch die Laserbehandlung hydrophile Oberflächen geschaffen werden, die sich gegebenenfalls durch eine chemische Nachbehandlung weiter verbessern lassen.

Die solchermaßen geschaffenen Lanzetten 10 können orientiert in einen Kunststoffhalter eingebracht werden. Dieser kann z.B. von einer Rolle kommen, und durch Einklipsen, Warmverformen etc. kann die Verbindung zur Lanzette lagerichtig erfolgen. Der Kunststoffhalter kann ein Kopplungsstück aufweisen, damit sich die Lanzetten im Gerät 18 greifen und bewegen lassen.

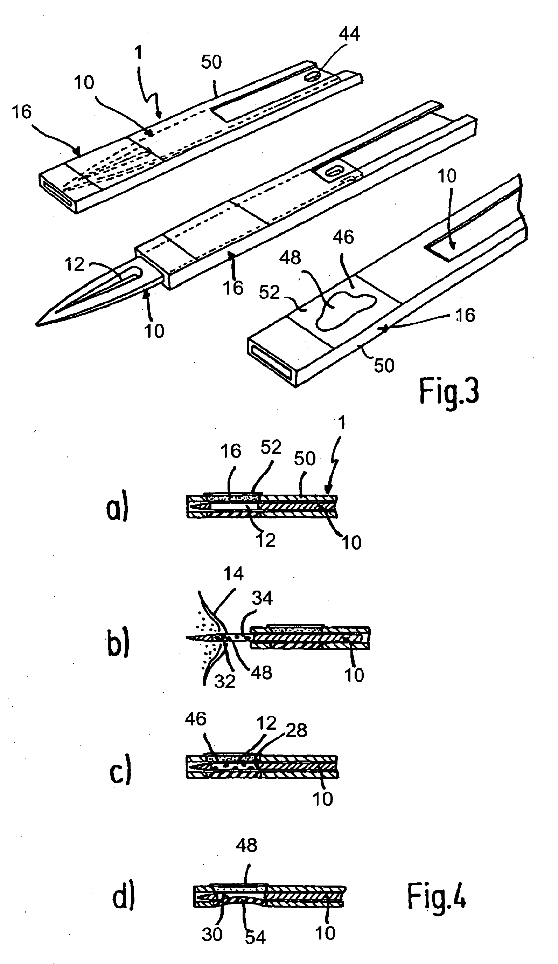

Fig. 3 zeigt ein kombiniertes System aus Probenentnahmeelement (Stechelement 10) und Probenempfangselement 16. Die Probenentnahme erfolgt durch hin- und hergehende Stechbewegung des Stechelements 10, wobei an dem proximalen Schaftteil 44 ein geeigneter Antrieb formschlüssig angekoppelt wird. Das während des Stechvorgangs gerätefest gehaltene Probenempfangselement 16 ist Teil einer Schubführung für das Stechelement 10 und weist ein Testfeld 46 zum Aufnehmen der zuvor in dem Längsschlitz 12 gesammelten Körperflüssigkeit 48 auf. Das Stechelement 10 erfüllt somit eine Zubringerfunktion als "Shuttle" bei der Probengewinnung, während der eigentliche Nachweis des Analyten auf dem mit dem Körperteil 14 nicht in Berührung kommenden Probenempfangselement 16 erfolgt. Dieses kann auch als Komponente eines eine Mehrzahl von abgeteilten Kompartimenten 50 umfassenden Magazins vorgesehen sein, welches sich als Verbrauchsartikel in das Gerät 18 einsetzen und mit den verbrauchten Stechelementen wieder entsorgen lässt. Der Glucosenachweis auf dem Testfeld bzw. Reagenzträger 46 kann photometrisch über Reflexion durch das transparente Deckfenster 52 hindurch erfolgen, so dass die Blutflüssigkeit 48 in dem Kompartiment 50 von den Geräteteilen hygienisch getrennt bleibt. Denkbar sind auch andere Nachweistechniken, beispielsweise über Flüoreszenz oder elektrochemische Erfassung.

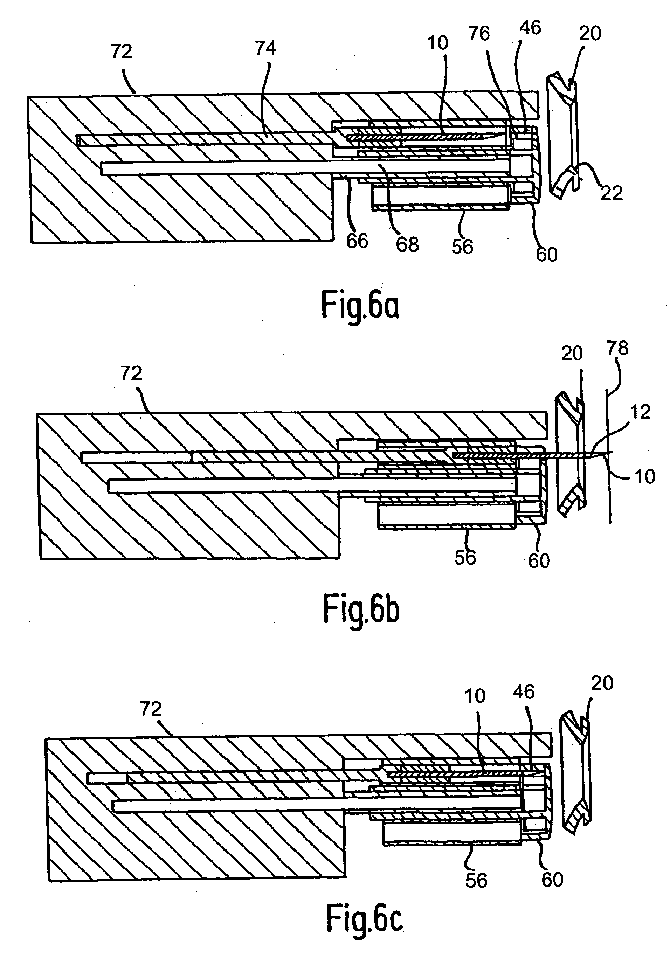

In Fig. 4 sind die wesentlichen Schritte des Probentransfers nochmals näher veranschaulicht. In der Ausgangsstellung (4a) befindet sich das Stechelement 10 geschützt in dem Magazinfach 50, welches frontseitig durch eine Durchstechfolie versiegelt sein kann. Die Einstichbewegung in das Körperteil 14 (beispielsweise eine Fingerkuppe) erfolgt aus Schmerzgründen möglichst rasch, wobei gegebenenfalls aus der maximalen Einstechtiefe eine leicht zurückgezogene Sammelposition angesteuert werden kann. Vorteilhafterweise ragt für den Sammelvorgang entsprechend (4b) der distale Schlitzabschnitt 32 in das Hautinnere, während der proximale Schlitzabschnitt 34 außerhalb davon eine Entlüftungsfunktion erfüllt. Somit kann die im Einstichkanal gewonnene Blutflüssigkeit in kurzer Sammelzeit beidseitig in den Sammelschlitz 12 einströmen und effizient das gesamte Schlitzvolumen füllen. In der Sammelstellung ist der Schlitz 12 von dem Probenempfangselement 16 räumlich so getrennt, dass keine fluidische Verbindung dazwischen besteht und die Nachweisreagenzien nicht in das Körperinnere gelangen können. Dies erlaubt es auch, den Nachweisvorgang gezielt zu starten und den Messverlauf als solchen auszuwerten. Zu diesem Zweck wird entsprechend Fig. 4c) das Stechelement 10 wieder zurückgezogen, bis die obere Seitenöffnung 28 des Schlitzes 12 unter das Testfeld 46 gelangt. Dieses kann beispielsweise durch eine vliesstruktur eine größere Kapillarattraktivität als der Schlitz 12 für einen selbsttätigen Transfer des aufgenommenen Blutes besitzen. Bevorzugt wird jedoch der Flüssigkeitstransfer unterstützt, indem gemäß Fig. 4d) an der unteren Schlitzöffnung 30 eine Membran 54 als Verdrängungsmittel für die gesammelte Flüssigkeit eingedrückt wird. Der Fluidtransfer erfolgt also über die gesamte Schlitzlänge mit kurzem Weg quer zur Stechrichtung des Stechelements 10.
Auch die in Fig. 5 bis 7 gezeigte Ausführungsform verwirklicht dieses Prinzip, wobei eine spezielle Magazinierung und Aktuierung vorgesehen ist. Gemäß Fig. 5 sind die Stechelemente 10 in einer Lanzettentrommel 56 magaziniert, an deren hinterer Stirnseite die Kopplungsstücke 44 für die Antriebskopplung abstehen, während die vordere (distale) Stirnseite durch eine Durchstechfolie 58 versiegelt wird. Eine gesonderte Teststreifentrommel 60 kann über Einschubschlitze mit Teststreifen 46 bestückt werden, welche dann durch eine Folie 62 und einen Stirndeckel 64 gegenüber der Umgebung insbesondere gegen Feuchtigkeitszutritt abgeschirmt werden. Der Stirndeckel 64 sitzt auf einer Trommelhohlachse 66, welche zugleich eine Luftpassage 68 für eine pneumatische Aktuierung über die mantelseitige Blasöffnung 70 bildet. Durch die gesonderte Magazinierung kann unterschiedlichen Anforderungen Sorge getragen werden. Die mit dem Körper in Kontakt kommenden Stechelemente 10 lassen sich in der Lanzettentrommel 56 zweckmäßig durch energiereiche Strahlung sterilisieren, während die strahlungsempfindliche Testchemie unabhängig davon in der Teststreifentrommel 60 gegen äußere Einflüsse geschützt bleibt und beim Stechvorgang auch nicht in Körperberührung gelangt.

Wie aus Fig. 6 ersichtlich, sind die Magazine 56, 60 im Einbauzustand auf der Trommelachse 66 axial gekoppelt, so dass jeweils ein Stechelement 10 und ein Teststreifen 46 einander zugeordnet sind. Eine Magazinaufnahme 72 innerhalb des Geräts 18 ermöglicht die Druckluftbeaufschlagung der Luftpassage 68 und den Stechantrieb des jeweils an einen Antriebsstößel 74 angekoppelten Stechelements 10 in koaxialer Ausrichtung zu dem Fingerkonus 20 (Fig. 6a). Beim Stechvorschub durchstößt das betätigte Stechelement 10 die vorgelagerte Kammer 76 des zugeordneten Teststreifens 46 und erreicht durch die Konusöffnung 22 hindurch die maximale Vorschubposition (Linie in Fig. 6b) bei einem zurückgelegten Weg von etwa 10 mm. Die Blutaufnahme über den Schlitz 12 erfolgt dann wie oben beschrieben. Anschließend wird bei der Rückbewegung des Stechelements 10 die in Fig. 6c gezeigte Transferposition angefahren, in welcher der Schlitz 10 in seiner Durchlassrichtung mit dem Teststreifen 46 fluchtet.

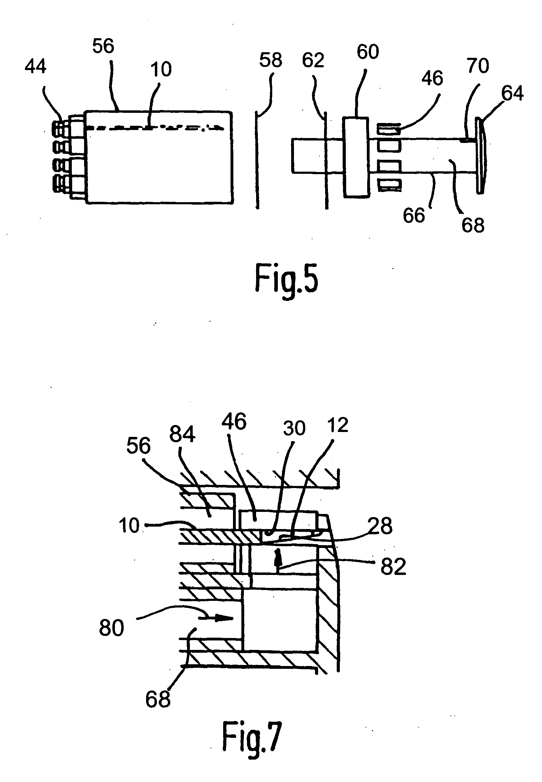

Wie am besten aus dem vergrößerten Ausschnitt gemäß Fig. 7 ersichtlich, ist in der Transferposition eine gezielte Übergabe der gesammelten Flüssigkeit auf den Teststreifen 46 möglich. Zu diesem Zweck wird über die Luftpassage 68 in Richtung der Pfeile 80, 82 ein Luftstoß ausgelöst, welcher den Schlitz 12 an der zugewandten Seitenöffnung 28 beaufschlagt, so dass die Blutflüssigkeit über die gegenüberliegende Seitenöffnung 30 auf den Teststreifen 46 verdrängt wird. Der Glucosenachweis kann dann über eine nicht gezeigte Geräteoptik reflektometrisch durchgeführt werden. Anschließend wird das kontaminierte Stechelement 10 in die Aufnahmekammer 84 des Trommelmagazins 56 vollständig zurückgezogen und durch Trommeldrehung das nächste Funktionspaar 10, 46 in Bereitschaft gebracht.

## Patentansprüche

1. Anordnung zum Aufnehmen von Körperflüssigkeiten wie Blut, mit einem vorzugsweise mit einem Stechorgan zum Einstechen in ein Körperteil (14) versehenen Probenentnahmeelement (10), das einen Sammelbereich (12) zum Sammeln von durch einen Einstich erhaltener Körperflüssigkeit aufweist, **dadurch gekennzeichnet, dass** der Sammelbereich durch einen als Kapillare langgestreckten, über Seitenöffnungen (28,30) an dem Probenentnahmeelement (10) beidseitig offenen Längsschlitz (12) gebildet ist.

2. Anordnung nach Anspruch 1, **dadurch gekennzeichnet, dass** die Länge des Längsschlitzes (12) so bemessen ist, dass in einer Sammelstellung des Probenentnahmeelements (10) der Längsschlitz (12) teilweise innerhalb und teilweise außerhalb des Körperteils (14) ist.

3. Anordnung nach Anspruch 1 oder 2, **dadurch gekennzeichnet, dass** der Längsschlitz (12) beim Sammeln der Körperflüssigkeit einen in die Haut des Körperteils (14) hineinragenden distalen Aufnahmeabschnitt (32) und einen außerhalb der Haut befindlichen proximalen Entlüftungsabschnitt (34) aufweist.

4. Anordnung nach einem der Ansprüche 1 bis 3, **dadurch gekennzeichnet, dass** der Längsschlitz (12) eine Länge von 0,5 bis 4 mm, vorzugsweise von 1 bis 2 mm aufweist.

5. Anordnung nach einem der Ansprüche 1 bis 4, **dadurch gekennzeichnet, dass** die Breite des Längsschlitzes (12) weniger als 500 µm, vorzugsweise weniger als 100 µm beträgt.

6. Anordnung nach einem der Ansprüche 1 bis 5, **dadurch gekennzeichnet, dass** der Längsschlitz (12) im Abstand vorzugsweise von etwa 50 bis 200 µm zu einer das Stechorgan bildenden distalen Spitze (26) des Probenentnahmeelements (10) angeordnet ist.

7. Anordnung nach einem der Ansprüche 1 bis 6, **dadurch gekennzeichnet, dass** das Probenentnahmeelement (10) im Bereich des Längsschlitzes (12) einen im Querschnitt verjüngten distalen Schaftabschnitt (40) und einen im Querschnitt erweiterten proximalen Schaftabschnitt (42) aufweist.

8. Anordnung nach einem der Ansprüche 1 bis 7, **gekennzeichnet durch** ein über den Sammelbereich (12) mit Körperflüssigkeit beaufschlagbares, vorzugsweise mit einem Testfeld (46) für einen Analyten in der Körperflüssigkeit versehenes Probenempfangselement (16) .

9. Anordnung nach Anspruch 8, **dadurch gekennzeichnet, dass** eine Seitenöffnung (28,30) als Auslass des Längsschlitzes (12) mit dem Probenempfangselement (16) in fluidischen Kontakt bringbar ist.

10. Anordnung nach Anspruch 8 oder 9, **dadurch gekennzeichnet, dass** der Längsschlitz (12) in der Sammelstellung des Probenentnahmeelements (10) von dem Probenempfangselement (16) getrennt und in einer Transferstellung des Probenentnahmeelements (10) mit dem Probenempfangselement (16) über eine Seitenöffnung (28,30) gekoppelt ist.

11. Anordnung nach einem der Ansprüche 8 bis 10, **dadurch gekennzeichnet, dass** die dem Auslass gegenüberliegende Seitenöffnung (28,30) des Längsschlitzes (12) in einer Transferstellung an einen Aktuator (54;68) zum Transfer der Körperflüssigkeit auf das Probenempfangselement (16) angeschlossen ist.

12. Anordnung nach Anspruch 11, **dadurch gekennzeichnet, dass** der Aktuator (54;68) über pneumatische und/oder mechanische Verdrängungsmittel auf die in dem Längsschlitz (12) befindliche Körperflüssigkeit einwirkt.

13. Anordnung nach Anspruch 11 oder 12, **dadurch gekennzeichnet, dass** der Aktuator durch eine gegen den Längsschlitz (12) deformierbare Membran (54) gebildet ist.

14. Anordnung nach einem der Ansprüche 11 bis 13, **dadurch gekennzeichnet, dass** der Aktuator eine an eine Seitenöffnung (28,30) des Längsschlitzes ankoppelbare Druckluftpassage (68) aufweist.

15. Anordnung nach einem der Ansprüche 8 bis 14, **dadurch gekennzeichnet, dass** das Probenempfangselement (16) eine größere Kapillarattraktion für die Körperflüssigkeit als der Längsschlitz (12) aufweist.

16. Anordnung nach einem der Ansprüche 8 bis 15, **dadurch gekennzeichnet, dass** das Probenentnahmeelement (10) in einer Führung (50) relativ zu dem Probenempfangselement (16) beweglich gelagert ist.

17. Anordnung nach einem der Ansprüche 8 bis 16, **dadurch gekennzeichnet, dass** mehrere Probenentnahmeelemente (10) in einem ersten Magazin (56) und mehrere Probenempfangselemente (16;46) in einem zweiten Magazin (60) bevorratet sind, wobei die Magazine (56,60) als gesonderte Einheiten zur paarweisen Kopplung der Probenentnahmeelemente und Probenempfangselemente miteinander verbindbar sind.

18. Anordnung nach einem der Ansprüche 8 bis 17, **dadurch gekennzeichnet, dass** eine Mehrzahl von Probenentnahmeelementen (10) in einem Magazin (56), vorzugsweise einem Trommelmagazin axial ausstoßbar angeordnet sind, und dass zugeordnete Probenempfangselemente (46) vorzugsweise in Durchstoßkammern (76) dem Magazin (56) in Ausstoßrichtung vorgeordnet sind.

19. Tragbares Blutanalysegerät mit mindestens einer vorzugsweise als Einmalartikel ausgebildeten Anordnung (1) nach einem der vorhergehenden Ansprüche.

20. Verfahren zur Herstellung eines Probenentnahmeelements (10) zum Aufnehmen von Körperflüssigkeiten wie Blut, insbesondere für eine Anordnung (1) nach einem der Ansprüche 1 bis 18, bei welchem durch Laserbearbeitung eine Kontur erzeugt wird, **dadurch gekennzeichnet, dass** in dem Probenentnahmeelement (10) ein beidseitig offener Längsschlitz (12) als Sammelbereich (12) für die Körperflüssigkeit durch Laserschneiden gebildet wird.

21. Verfahren nach Anspruch 20, **dadurch gekennzeichnet, dass** die Begrenzungsfläche des Längsschlitzes (12) beim Laserschneiden hydrophiliert wird.

22. Verfahren nach Anspruch 20 oder 21, **dadurch gekennzeichnet, dass** die Laserenergie und/oder die Verfahrgeschwindigkeit des Laserstrahls beim Laserschneiden positionsabhängig geregelt wird.

23. Verfahren nach einem der Ansprüche 20 bis 22, **dadurch gekennzeichnet, dass** die Bearbeitungszeit während des Laserschneidens weniger als 2 s, vorzugsweise etwa 1 s beträgt.

24. Verfahren nach einem der Ansprüche 20 bis 23, **dadurch gekennzeichnet, dass** die Begrenzungskanten des Längsschlitzes (12) mit einem Kantenwinkel von weniger als 100°, vorzugsweise etwa 90° gebildet werden.

25. Verfahren nach einem der Ansprüche 20 bis 24, **dadurch gekennzeichnet, dass** an dem Probenentnahmeelement (10) ein distales Stechorgan (26) durch Schleifen erzeugt wird, so dass das Stechorgan durch mindestens einen Flachschliff (36) begrenzt wird.

26. Verfahren nach einem der Ansprüche 20 bis 25, **dadurch gekennzeichnet, dass** ein Draht oder ein flaches Band als Ausgangsmaterial bearbeitet wird.
